(19) Europäisches Patentamt
European Patent Office
Office européen des brevets

(11) Veröffentlichungsnummer : **0 209 025**
**B1**

(12) # EUROPÄISCHE PATENTSCHRIFT

(45) Veröffentlichungstag der Patentschrift : **28.09.88**

(51) Int. Cl.⁴ : **C 07 C 83/04**, C 07 C135/00, A 23 K 1/16

(21) Anmeldenummer : **86109191.6**

(22) Anmeldetag : **04.07.86**

(54) **Arylethanolhydroxylamine, Verfahren zu ihrer Herstellung und ihre Verwendung zur Leistungsförderung.**

(30) Priorität : **16.07.85 DE 3525336**

(43) Veröffentlichungstag der Anmeldung : **21.01.87 Patentblatt 87/04**

(45) Bekanntmachung des Hinweises auf die Patenterteilung : **28.09.88 Patentblatt 88/39**

(84) Benannte Vertragsstaaten : **AT BE CH DE FR GB IT LI NL SE**

(56) Entgegenhaltungen :
**EP-A- 0 026 298**
**DE-A- 2 351 281**
**DE-B- 1 543 728**
**GB-A- 2 137 619**
**CHEMICAL ABSTRACTS, Band 90, Nr. 15, 9. April 1979, Columbus, Ohio, USA, S. JORDAN et al. "The synthesis and oxidation of N-hydroxy derivatives of the beta-adrenoceptor antagonists bufuralol and toliprolol", p. 618, Zusammenfassung-Nr. 121 317a**
**CHEMICAL ABSTRACTS, Band 98, Nr. 11, 14. März 1983, Columbus, Ohio, USA, S. ZHANG et al. "Derivatives of beta-adrenergic antagonists: N-nitro-sopropanolol and N-hydroxypropanolol and its aldonitrone", p. 516, Zusammenfassung-Nr. 88 940p**
**J.Chem.Soc., Perkin Trans. 1(9), 1978, p. 928-933**
**AUSTALIAN JOURNAL OF CHEMISTRY, Band 31, September 1978, Melbourne, D.S.C. BLACK et al. "Nitrones and oxaziridines. XX. Intramolecular nitrone 1,3-dipolar cycloaddition by substituent interaction: synthesis and reduction of some tetrahydropyrroloisaxazoles", pp. 2013-2022**

(73) Patentinhaber : **BAYER AG**
**Konzernverwaltung RP Patentabteilung**
**D-5090 Leverkusen 1 Bayerwerk (DE)**

(72) Erfinder : **Ingendoh, Axel, Dr.**
**Engstenberger Hoehe 1o**
**D-5068 Odenthal-Osenau (DE)**
Erfinder : **Lindel, Hans, Dr.**
**Carl-Duisberg-Strasse 321**
**D-5090 Leverkusen 1 (DE)**
Erfinder : **Berschauer, Friedrich, Dr.**
**Claudiusweg 9**
**D-5600 Wuppertal 1 (DE)**
Erfinder : **De Jong, Anno, Dr.**
**Stockmannsmühle 46**
**D-5600 Wuppertal 1 (DE)**
Erfinder : **Scheer, Martin, Dr.**
**Herberts-Katernberg 7**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft Arylethanolhydroxylamine, Verfahren zu ihrer Herstellung und ihre Verwendung als Leistungsförderer für Tiere.

Arylethanolamine sind bekannte Verbindungen. Sie besitzen in Abhängigkeit von ihrer chemischen Struktur unterschiedliche pharmakologische Eigenschaften. Unter anderem besitzen bestimmte Arylethanolamine Wirkungen auf die Gewichtszunahme von Tieren sowie auf das Verhältnis von Fleisch zu Fett (EP-OS-26 298). Auch dabei scheint die Grundstruktur des Arylethanolamins von entscheidender Bedeutung für die Wirkung zu sein.

Es wurden die neuen Arylethanolhydroxylamine der Formel I

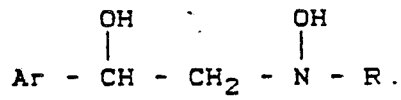

(I)

in welcher

Ar für Aryl, das gegebenenfalls substituiert sein kann, steht,

R für Alkyl, Aryl, Aralkyl, Heteroaralkyl, Heteroaryl, Cycloalkyl, die gegebenenfalls substituiert sein können, steht,

sowie deren physiologisch verträgliche Salze, Enantiomeren und Diastereomeren gefunden.

Es wurde ein Verfahren zur Herstellung der Arylethanolhydroxylamine der Formel I

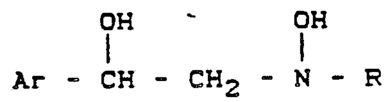

(I)

in welcher

Ar für Aryl, das gegebenenfalls substituiert sein kann, steht,

R für Alkyl, Aryl, Aralkyl, Heteroaralkyl, Heteroaryl, Cycloalkyl, die gegebenenfalls substituiert sein können, steht,

gefunden, das dadurch gekennzeichnet ist, daß man Arylglyoxalnitrone der Formel II

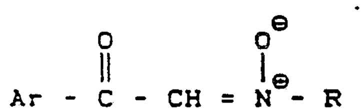

(II)

in welcher

Ar und R die oben angegebene Bedeutung haben, reduziert.

Es wurden die neuen Arylglyoxalnitrone der Formel II gefunden

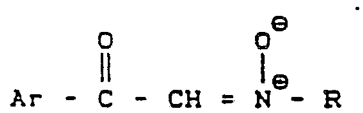

(II)

in welcher

Ar für Aryl, das gegebenenfalls substituiert sein kann, mit Ausnahme von Phenyl und p-Bromphenyl steht und

R für Alkyl, Aralkyl, Heteroaralkyl, Cycloalkyl, die gegebenenfalls substituiert sein können, steht.

Es wurde ein Verfahren zur Herstellung der neuen Arylglyoxalnitrone der Formel II

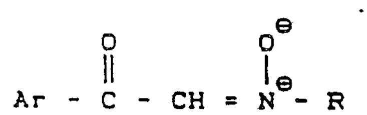

(II)

in welcher

Ar für Aryl, das gegebenenfalls substituiert sein kann, mit Ausnahme von Phenyl und p-Bromphenyl steht und

R für Alkyl, Aralkyl, Heteroaralkyl, Cycloalkyl, die gegebenenfalls substituiert sein können, steht.

gefunden, das dadurch gekennzeichnet ist, daß man Arylglyoxale der Formel III

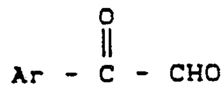

in welcher

Ar die oben angegebene Bedeutung hat,

gegebenenfalls in Form ihrer Hydrate oder Addukte mit Alkoholen mit Hydroxylaminen der Formel IV

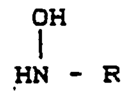

in welcher

R die oben angegebene Bedeutung hat, unter Wasserabspaltung umsetzt.

Die Arylethanolhydroxylamine zeigen hervorragende leistungssteigernde Wirkung bei Tieren. Sie lassen sich daher auf dem Gebiet der Tierernährung als Leistungsförderer einsetzen. Diese Eigenschaft war erstaunlich. Es war nämlich bei Arylethanolaminen bekannt, daß Änderungen an der Substitution des Aminteils des Moleküls zu starken Änderungen der biologischen und pharmakologischen Wirkung führen. Es konnte daher keinesfalls erwartet werden, daß eine völlig andere chemische Klasse von Verbindungen, nämlich Hydroxylamine, wirtschaftlich verwertbare Wirkungen aufweisen.

Bevorzugt sind Arylethanolhydroxylamine der Formel I,

in welcher

Ar für Phenyl, Naphthyl, steht, die gegebenenfalls substituiert sein können durch einen oder mehrere gleiche oder verschiedene Substituenten aus der Gruppe Alkyl, Halogenalkyl, Aryl, Alkoxy, Halogenalkoxy, Halogen, Nitro, Hydroxy, Cyano, Alkoxyimino, Alkylcarbonyl, Amino, Aminocarbonyl, Dialkylaminocarbonyl, Alkyl- und Dialkylamino, Alkylaminocarbonyl, Aminosulfonyl, Alkylsulfonylamino, Alkylsulfonyl, Alkylsulfinyl, Alkylthio, Halogenalkylthio, Alkoxycarbonylamino, Aminocarbonylamino, Alkylendioxy, Halogenalkylendioxy, gegebenenfalls substituiertes Aryl, Aryloxy, Arylthio,

und

R für geradkettiges oder verzweigtes Alkyl, Cycloalkyl, Bicycloalkyl mit bis zu 12 C-Atomen, die gegebenenfalls substituiert sein können durch Alkinyl, Alkenyl, Alkoxy, Alkylthio, Hydroxy, Halogen, gegebenenfalls substituiertes Aryl, gegebenenfalls substituiertes Heteroaryl oder Aryloxy steht.

Als Substituenten der gegebenenfalls substituierten Reste kommen bevorzugt in Frage : Cyano, Halogen wie Fluor, Chlor, Brom, Hydroxy, $C_{1-4}$-Alkyl, $C_{1-4}$-Halogenalkyl, Phenyl, $C_{1-4}$-Alkoxy, $C_{1-4}$-Halogenalkoxy, $C_{1-4}$-Alkylthio, $C_{1-4}$-Halogenalkylthio. Für den Fall, daß die Substituenten an einem Phenylrest sitzen, zusätzlich bevorzugt sind Methylendioxy, Ethylendioxy, halogensubstituiertes Methylendioxy, halogensubstituiertes Ethylendioxy, ferner Phenyl Phenoxy, die ihrerseits einen oder mehrere der obengenantnen Substituenten tragen können.

Besonders bevorzugt sind Verbindungen der Formel I, in welcher

Ar für Phenyl steht, das ein- oder mehrfach substituiert ist durch gleiche oder verschiedene Reste aus der Gruppe $C_{1-4}$-Alkyl, Phenyl, $C_{1-4}$-Alkoxy, Halogen, insbesondere Fluor, Chlor, Brom, Cyano, Nitro, $C_{1-4}$-Halogenalkyl, Amino, $C_{1-4}$-Alkylamino, Di-($C_{1-4}$)-alkylamino, $C_{1-4}$-Alkoxycarbonyl, Aminocarbonyl, $C_{1-4}$-Alkylaminocarbonyl, Di-($C_{1-4}$)-alkylaminocarbonyl, Aminosulfonyl, $C_{1-4}$-Alkylaminosulfonyl, Di-($C_{1-4}$)-alkylaminosulfonyl, $C_{1-4}$-Alkylsulfonylamino, $C_{1-4}$-Alkylsulfonyl, $C_{1-4}$-Alkylsulfinyl, $C_{1-4}$-Alkylthio, $C_{1-4}$-Alkoxycarbonylamino und Hydroxy, $C_{1-4}$-Halogenalkoxy, $C_{1-4}$-Halogenalkylthio, Methylendioxy, Ethylendioxy, Halogenmethylendioxy, Phenoxy, das seinerseits wieder durch einen der genannten Reste substituiert sein kann,

R für geradkettiges oder verzweigtes Alkyl, Cycloalkyl oder Bicycloalkyl mit bis zu 12 C-Atomen steht, die gegebenenfalls substituiert sind durch $C_{2-4}$-Alkinyl, $C_{2-4}$-Alkenyl, Hydroxy, Halogen, wie Fluor, Chlor, Brom, Phenyl, gegebenenfalls substituiert durch insbesondere Halogen, $C_{1-4}$-Alkoxy, $C_{1-4}$-Alkyl und Nitro.

Besonders bevorzugt sind ferner Verbindungen der Formel I,

in welcher

Ar für Phenyl steht, das durch 1, 2 oder 3 gleiche oder verschiedene Reste aus der Gruppe Phenyl, $C_{1-4}$-Alkyl, $C_{1-4}$-Halogenalkyl, Cyano, Hydroxy, Amino, $C_{1-4}$-Alkyl und Di-$C_{1-4}$-alkylamino, Halogen und Nitro substituiert ist und

R für geradkettiges oder verzweigtes Alkyl oder Cycloalkyl mit bis zu 8 C-Atomen steht, das gegebenenfalls substituiert ist durch $C_{2-4}$-Alkinyl, $C_{2-4}$-Alkenyl, Hydroxy, Halogen, gegebenenfalls durch insbesondere Methoxy, $C_{1-4}$-Alkyl, Nitro, $CF_3$, Halogen, wie Chlor oder Fluor substituiertes Phenyl.

Insbesondere seien Verbindungen der Formel I genannt, in denen

Ar für Phenyl steht, das ein- oder mehrfach, gleich oder verschieden, durch Fluor, Chlor, Brom, Amino, und CN substituiert ist und

R für $C_{1-5}$-Alkyl steht, das gegebenenfalls substituiert ist durch $C_{3-6}$-Cycloalkyl, insbesondere Cyclopropyl, Ethinyl, Hydroxy, Halogen, Phenyl, das seinerseits gegebenenfalls durch Halogen wie Fluor, Chlor, Trifluormethyl, Methoxy substituiert ist.

Im einzelnen seien neben den in den Beispielen genannten Verbindungen folgende Verbindungen

der Formel I genannt :

| Ar | R |
|---|---|
| 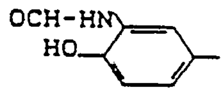 | $C(CH_3)_3$ |
| 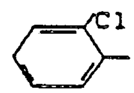 | $C(CH_3)_3$ |
| 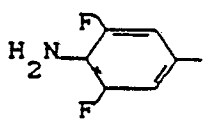 | $C(CH_3)_3$ |
| 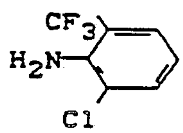 | $C(CH_3)_3$ |

Die Verbindungen der Formel I können auch in Form ihrer Enantiomere oder, falls mehrere optisch aktive C-Atome im Molekül vorliegen, in Form ihrer Diastereomere vorliegen.

Physiologisch verträgliche Salze der Verbindungen der Formel I können mit folgenden Säuren gebildet werden.

Salzsäure, Schwefelsäure, Phosphorsäure, Perchlorsäure, Brom-, Iod-, Fluorwasserstoffsäure, Salpetersäure, Essigsäure, Oxalsäure, Malonsäure, Bernsteinsäure, Ascorbinsäure, Apfelsäure, Weinsäure, Maleinsäure, Fumarsäure, Methansulfonsäure, Benzoesäure, substituierte Benzoesäure, Ameisensäure, Chloressigsäure, Toluolsulfonsäure, Benzolsulfonsäure, Trichloressigsäure, Phthalsäure, Naphthalinsulfonsäure, Nikotinsäure.

Die Verbindungen der Formel I werden erhalten, indem man Arylglyoxalnitrone der Formel II reduziert. Die Reaktion läßt sich durch folgendes Formelschema wiedergeben :

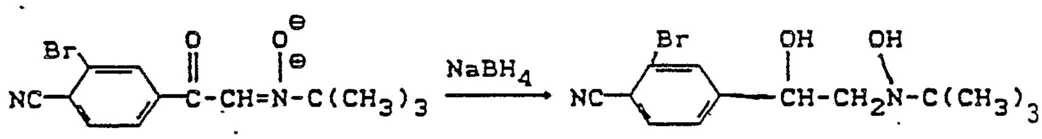


Arylglyoxalnitrone der Formel II sind teilweise (vgl. D. Black et al Aust. J. Chem. (1978) 31, 2013-22) neu. Ihre Herstellung wird weiter unten beschrieben. Es werden bevorzugt die Arylglyoxalnitrone der Formel II eingesetzt, in der die Reste Ar und R die bei den Verbindungen der Formel I angegebenen bevorzugten Bedeutungen haben.

Die Reaktion erfolgt bevorzugt mit folgenden Reduktionsmitteln : komplexe Hydride, wie z. B. Alkaliborhydride wie z. B. $NaBH_4$, $NaBH_3CN$, $LiBH_4$, $NaBH_x$ (O-Alkyl)$_y$, wobei x und y sich zu 4 ergänzen. Besonders bevorzugt ist dabei $NaBH_4$.

Die Umsetzung erfolgt bevorzugt in folgenden Verdünnungsmitteln :

Inerte organische Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Alkohole, wie Methanol, Ethanol, Isopropanol oder höherkettige Alkohole, außerdem Ester, wie Essigsäuremethylester und -ethylester, ferner Nitrile, wie z. B. Acetonitril und Propionitril, Benzonitril, Glutarsäuredinitril, darüber hinaus Amide, wie z B. Dimethylformamid, Dimethylacetamid und N-Methylpyrrolidon, sowie Dimethylsulfoxid, Tetramethylensulfon und Hexamethylphosphorsäuretriamid.

Die Reaktion erfolgt bei Temperaturen von —50-100 °C, vorzugsweise bei Temperaturen zwischen 0 °C und 50 °C.

Die Aufarbeitung erfolgt in üblicher Weise.

Arylglyoxalnitrone der Formel II sind neu. Sie werden erhalten, indem man Arylglyoxale der Formel III mit N-Alkylhydroxylaminen der Formel IV umsetzt. Die Reaktion läßt sich durch folgendes Formelschema wiedergeben :

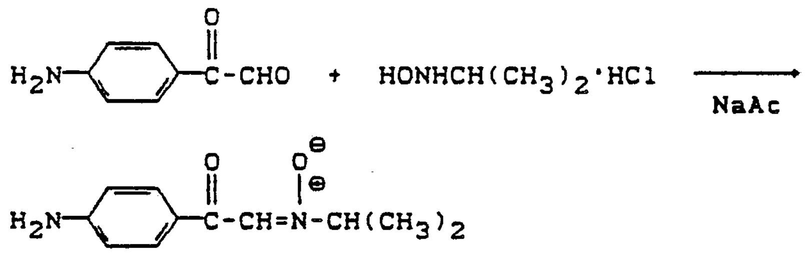


Arylglyoxale der Formel III sind teilweise bekannt. Neue Arylglyoxale lassen sich nach an sich bekannten Verfahren herstellen, indem man z. B. die entsprechenden α-Halogenacetophenone mit DMSO oxidiert oder Acetophenone mit Selendioxid oxidiert.

Es werden bevorzugt Arylglyoxale der Formel III eingesetzt, in der Ar die bei den Verbindungen der Formel I angegebenen bevorzugten Bedeutungen hat.

Im einzelnen seien folgende Arylglyoxale genannt :

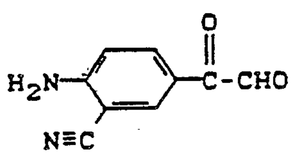 ; 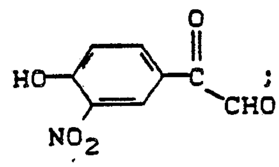 ; 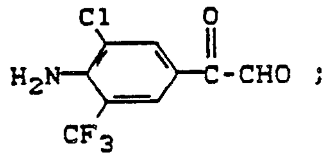 ;

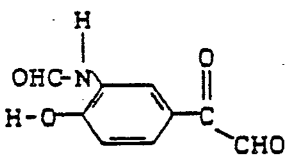 ; 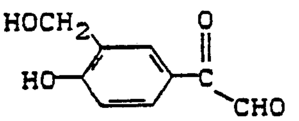 ; 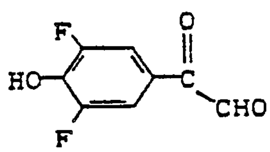

Hydroxylamine der Formel IV sind bekannt oder lassen sich nach an sich bekannten Methoden herstellen. Es werden bevorzugt Hydroxylamine der Formel IV eingesetzt, in der R die bei den Verbindungen der Formel I angegebenen bevorzugt Bedeutungen hat.

Die Umsetzung kann mit oder ohne Verdünnungsmittel erfolgen. Als Verdünnungsmittel seien genannt :

Alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol, und o-Dichlorbenzol, Alkohole wie Methanol, Ethanol, Isopropanol, Glycol und höherkettige Alkohole, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z. B. Acetonitril und Propionitril, Benzonitril, Glutarsäuredinitril, darüber hinaus Amide, wie z. B. Dimethylformamid, Dimethylacetamid und N-Methylpyrrolidon, sowie Dimethylsulfoxid, Tetramethylensulfon und Hexamethylphosphorsäuretriamid.

Vorzugsweise setzt man das N-Alkylhydroxylamin der allgemeinen Formel IV als Salz einer anorganischen oder organischen Säure mit dem Arylglyoxal der allgemeinen Formel III in Gegenwart eines Alkalisalzes einer schwachen organischen Säure wie z. B. Natriumacetat um.

Die Reaktion kann bei Temperaturen von 10-200 °C, bevorzugt von 50-150 °C, ganz besonders bevorzugt bei der Siedetemperatur des Lösungsmittels, erfolgen.

Es wird bevorzugt in Gegenwart eines wasserentziehenden Mittels gearbeitet. Als solche seien genannt z. B. $Na_2SO_4$, $MgSO_4$, $K_2CO_3$, $CaCl_2$, Molekularsiebe, Kieselgel, Aluminiumoxide. Das bei der Reaktion entstehende Wasser kann auch azeotrop aus dem Reaktionsgemisch entfernt werden.

Die Verbindungen der Formel I werden bevorzugt im äquimolaren Verhältnis eingesetzt.

Die Aufarbeitung erfolgt in an sich bekannter Weise.

Es muß bei der Herstellung von Arylethanolhydroxylaminen der allgemeinen Formel I nicht in jedem Falle das Arylglyoxalnitron der allgemeinen Formel II isoliert werden. Es kann sich als besonders günstig erweisen, die Reaktionsfolge in einer Eintopfreaktion durchzuführen.

Die Wirkstoffe werden als Leistungsförderer bei Tieren zur Förderung und Beschleunigung des Wachstums, der Milch- und Wollproduktion, sowie zur Verbesserung der Futterverwertung, der

Fleischqualität und zur Verschiebung des Fleisch-Fett-Verhältnisses zugunsten von Fleisch eingesetzt. Die Wirkstoffe werden bei Nutz-, Zucht-, Zier- und Hobbytieren verwendet.

Zu den Nutz- und Zuchttieren zählen Säugetiere wie z. B. Rinder, Schweine, Pferde, Schafe, Ziegen, Kaninchen, Hasen, Damwild, Pelztiere wie Nerze, Chinchilla, Geflügel wie z. B. Hühner, Gänse, Enten, Truthähne, Tauben, Fische wie z. B. Karpfen, Forellen, Lachse, Aale, Schleien, Hechte, Reptilien wie z..B. Schlangen und Krokodile.

Zu den Zier- und Hobbytieren zählen Säugetiere wie Hunde und Katzen, Vögel wie Papageien, Kanarienvögel, Fische wie Zier- und Aquarienfische z. B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, der Wachstums- und Leitungsphase, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstums- und Leistungsphase temporär oder kontinuierlich verabreicht werden.

Bei kontinuierlicher Verabreichung kann die Anwendung ein- oder mehrmals täglich in regelmäßigen oder unregelmäßigen Abständen erfolgen.

Die Verabreichung erfolgt oral oder parenteral in dafür geeigneten Formulierungen oder in reiner Form.

Die Wirkstoffe können in den Formulierungen allein oder in Mischung mit anderen leistungsfördernden Wirkstoffen, mineralischen Futtermitteln, Spurenelement-Verbindungen, Vitaminen, Nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind : z. B. Antibiotika wie Tylosin und Virginiamycin. Mineralische Futtermittel sind z. B. Dicalciumphosphat, Magnesiumoxid, Natriumchlorid.

Spurenelement-Verbindungen sind z. B. Eisenfumarat, Natriumjodid, Kobaltchlorid, Kupfersulfat, Zinkoxid. Vitamine sind z. B. Vitamin A, Vitamin $D_3$, Vitamin E, B-Vitamine, Vitamin C.

Nicht-Protein-Verbindungen sind z. B. Biuret, Harnstoff. Farbstoffe sind z. B. Carotinoide wie Citranaxanthin, Zeaxanthin, Capsanthin.

Antioxidantien sind z. B. Aethoxyquin, Butylhydroxy-Toluol.

Aromastoffe sind z. B. Vanillin.

Emulgatoren sind z. B. Ester der Milchsäure, Lecithin.

Fließhilfsstoffe sind z. B. Natriumstearat, Calciumstearat.

Konservierungsstoffe sind z. B. Zintronensäure, Propionsäure.

Preßhilfsstoffe sind z. B. Ligninsulfonate, Cellulose-ether.

Die Verabreichung der Wirkstoffe kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Herkunft wie Fleisch, Fette, Milchprodukte, Knochenmehl, Fischprodukte, die Einzelfuttermittel wie Vitamine, Proteine, Aminosäuren z. B. DL-Methionin, Salze wie Kalk und Kochsalz. Zum Futter zählen auch Ergänzungs-, Fertig- und Mischfuttermittel. Diese enthalten Einzelfuttermittel in einer Zusammensetzung, die eine ausgewogene Ernährung hinsichtlich der Energie- und Proteinversorgung sowie der Versorung mit Vitaminen, Mineralsalzen und Spurenelementen sicherstellt.

Die Konzentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das erfindungsgemäßen Wirkstoff enthält :

200 g Weisen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Prämix ergeben nach sorgfältigem Mischen 1 kg Futter.

In einem kg Futtermischung sind enthalten : 600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $MnSO_4 \times H_2O$, 140 mg $ZnSO_4 \times 7 H_2O$, 100 mg $FeSO_4 \times 7 H_2O$ und 20 mg $CuSO_4 \times 5 H_2O$.

2,5 g Wirkstoff-Prämix enthalten z. B. 10 mg Wirkstoff, 1 g DL-Methion, Rest Sojabohnenmehl.

Beispiel für die Zusammensetzung eines Schweineaufzuchtfutters, das erfindungsgemäßen Wirkstoff enthält :

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z. B. wie beim Kükenfutter) ergeben nach sorgfältigem Mischen 1 kg Futter.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

## Beispiel A

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Futterverbrauch bestimmt.

Es werden die aus der Tabelle ersichtlichen Ergebnisse erhalten :

Tabelle 1

Ratten Fütterungsversuch

| Wirkstoff Beispiel Nr. | Wirkstoff Aufwand ppm | Körpergewichtszunahme g während 13 Tagen |
|---|---|---|
| Kontrolle | 0 | 36,4 |
| 5 | 1 | 40,0 |
| | 25 | 47,3 |
| 4 | 25 | 45,5 |
| 14 | 25 | 45,1 |
| 27 | 25 | 43,0 |

## Beispiele

### Beispiel 1

N-t-Butyl-N-(2-hydroxy-2-phenyl-ethyl)-hydroxylamin

$$C_6H_5-CHOH-CH_2-\underset{\displaystyle OH}{\underset{|}{N}}-C(CH_3)_3$$

Zu 5 g N-t-Butyl-phenylglyoxalnitron in 150 ml Methanol gelöst, wurden bei Raumtemperatur portionsweise 1,8 g $NaBH_4$ zugegeben. Nach 3 bis 4 Stunden wurde einrotiert, der Rückstand in der Kälte mit 100 ml 10 %iger HCl versetzt, dreimal mit 25 ml Ether extrahiert und die wäßrige Phase unter Kühlung mit 120 ml 10 %iger wäßriger Natronlauge alkalisiert. Man extrahiert die wäßrige Phase mehrmals mit Dichlormethan, trocknet die organische Phase über Natriumsulfat und engte ein. Den Rückstand löste man in 50 ml Ether und versetzte mit 150 ml einer gesättigten etherischen Oxalsäure-Lösung. Der dabei entstehende Niederschlag des Oxalats wurde isoliert und umkristallisiert.

Ausbeute : 2,7 g. Schmp. : 143-144 °C.

Analog wurden folgende Verbindungen der Formel I erhalten

$$Ar-CHOH-CH_2-\underset{\displaystyle OH}{\underset{|}{N}}-R \qquad (I)$$

| Beispiel | Ar | R | Schmp.: |
|---|---|---|---|
| 2 | $C_6H_5$ | $-CH(CH_3)_2$ | 123° C (Oxalat) |
| 3 | $C_6H_5$ | $CH_3$ | 104° C (Oxalat) |
| 4 | $4-NH_2,3,5-Cl_2-C_6H_2-$ | $-CH(CH_3)_2$ | 148° C (Oxalat) |
| 5 | $4-NH_2,3,5-Cl_2-C_6H_2-$ | $-C(CH_3)_3$ | 95-100° C (Oxalat) |
| 6 | $4-NH_2,3,5-Cl_2-C_6H_2-$ | $-C(CH_3)_2C\equiv CH$ | 76-85° C (Oxalat) Zers. |
| 7 | $4-NH_2,3,5-Cl_2-C_6H_2-$ | $-CH(CH_3)-CH_2-2-CH_3O-C_6H_4$ | Oel |
| 8 | $4-NH_2,3,5-Cl_2-C_6H_2-$ | $-C(CH_3)-C_2H_5$ | 72-75° C (Oxalat) |
| 9 | $4-NH_2,3,5-Cl_2-C_6H_2-$ | $-CH(CH_3)-CH_2-2-F-C_6H_4$ | 90° C (Oxalat) Zers. |
| 10 | $4-NH_2,3,5-Cl_2-C_6H_2-$ | $-CH(CH_3)-CH_2-4-CH_3O-C_6H_4$ | 78° C (Oxalat) |
| 11 | $4-NH_2,3,5-Cl_2-C_6H_2-$ | $-CH(CH_3)-CH_2-3-CF_3-C_6H_4$ | Oel |
| 12 | $4-NH_2,3,5-Cl_2-C_6H_2-$ | $-CH(CH_3)-CH_2-3,4-CH_3O-C_6H_3$ | 115° C |
| 13 | $4-NH_2,3,5-Cl_2-C_6H_2-$ | $-CH_2-CH(CH_3)_2$ | Oel |
| 14 | $4-NH_2,3,5-Cl_2-C_6H_2-$ | $-C(CH_3)_2-CH_2OH$ | 85° C (Oxalat) |
| 15 | $4-NH_2,3,5-Cl_2-C_6H_2-$ | $-CH(CH_3)-CH_2-2-F,6-Cl-C_6H_3$ | 76° C (Oxalat) |

| Beispiel | Ar | R | Schmp.: |
|---|---|---|---|
| 16 | $4-NH_2,3,5-Cl_2-C_6H_2-$ | $-CH_2-C(CH_3)_3$ | $108^0$ C (Oxalat) |
| 17 | $4-NH_2,3,5-Cl_2-C_6H_2-$ | $-CH(CH_3)-CH_2-4-F-C_6H_4$ | Oel |
| 18 | $4-NH_2,3,5-Cl_2-C_6H_2-$ | $-CH_2-CH_2-$ 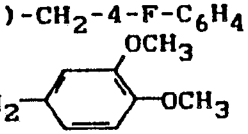 | Oel |
| 19 | $4-NH_2,3,5-Cl_2-C_6H_2-$ | $-CH(cyclo-C_3H_5)_2$ | Oel |
| 20 | $4-NH_2,3,5-Cl_2-C_6H_2-$ | $-CH(CH_3)-CH_2-3-Cl-C_6H_4$ | fest (Oxalat) |
| 21 | $4-NH_2,3,5-Cl_2-C_6H_2-$ | $-CH(CH_3)-CH_2-2,6-Cl_2-C_6H_3$ | fest (Oxalat) |
| 22 | $4-NH_2,3,5-Cl_2-C_6H_2-$ | 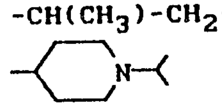 | fest (Oxalat) |
| 23 | $4-NH_2,3,5-Cl_2-C_6H_2-$ | 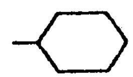 | Oxalat |
| 24 | $4-NH_2,3,5-Cl_2-C_6H_2-$ | 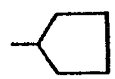 | Oxalat |
| 25 | $4-NH_2,3,5-Cl_2-C_6H_2-$ | $-nC_4H_9$ | Oxalat |
| 26 | $4-NH_2,3,5-Cl_2-C_6H_2-$ | $-CH(CH_3)C(CH_3)_3$ | Oxalat |
| 27 | $4-NH_2-$, $3-Cyano-C_6H_3-$ | $-CH(CH_3)_2$ | $51^0$ C |
| 28 | $4-NH_2-$, $3-Cyano-C_6H_3-$ | $-C(CH_3)_3$ | $59^0$ C |
| 29 | $4-NH_2-$, $3-Cyano-C_6H_3-$ | $-C(CH_3)_2-CH_2OH$ | Öl |

| Beispiel | Ar | R | Schmp.: |
|---|---|---|---|
| 30 | 4-OH-$C_6H_4$- | $C(CH_3)_3$ | Öl |
| 31 | 4-$NO_2$-$C_6H_4$- | $C(CH_3)_3$ | Öl |
| 32 | 2-Naphthyl | $C(CH_3)_3$ | Öl |
| 33 | 4-$NH_2$, 3,5-$CH_2$-$C_6H_2$- | $CH_3$ | 67° C |
| 34 | 4-Phenyl-$C_6H_4$- | $C(CH_3)_3$ | 69° C |
| 35 | 4-OH, 3-$NO_2$-$C_6H_3$- | $C(CH_3)_3$ | 69 (als Oxalat) |
| 36 | 4-$NH_2$, 3-Cl, 5-$CF_3$-$C_6H_2$- | $C(CH_3)_3$ | Öl |
| 37 | 4-$NH_2$, 3,5-$Cl_2$-$C_6H_2$- | $CH(CH_3)CF_3$ | 136 (als Oxalat) |
| 38 | 4-$NH_2$, 3-5-$Cl_2$-$C_6H_2$- | $CH_2$-$CF_3$ | Öl |
| 39 | 4-$NH_2$, 3,5-$Cl_2$-$C_6H_2$- | 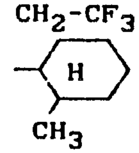 | Öl |
| 40 | 4-$NH_2$, 3,5-$Cl_2$-$C_6H_2$- | -$CH(CH_3)$-$CH_2$-OH | Öl |
| 41 | 4-$NH_2$, 3,5-$Cl_2$-$C_6H_2$- | -$CH(CH_3)$-$C_3H_7$ | Öl |
| 42 | 4-$NH_2$, 3,5-$Cl_2$-$C_6H_2$- | -$CH(C_2H_5)_2$ | Öl |
| 43 | 4-$NH_2$, 3,5-$Cl_2$-$C_6H_2$- | -$CH(CH_3)CH(CH_3)_2$ | Öl |
| 44 | 3,4-$Cl_2$-$C_6H_3$- | -$C(CH_3)_3$ | Öl |
| 45 | 4-$NH_2$, 3,5-$Cl_2$-$C_6H_2$- | 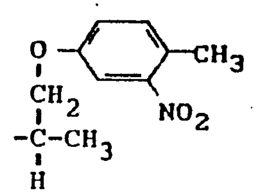 | 59-62 |

| Beispiel | Ar | R | Schmelzpunkt/°C |
|---|---|---|---|
| 46 | 4-OH, 3-$CH_3OCO$-$C_6H_3$- | -$C(CH_3)_3$ | 138-140 |
| 47 | 3,5-$Cl_2$, 4- $CH_3O$-$C_6H_2$- | -$C(CH_3)_3$ | 175-177 |
| 48 | 5-Cl, 4-$NO_2$-$C_6H_3$- | -$C(CH_3)_3$ | 94 |
| 49 | 3,5-$Cl_2$, 4-$NH_2$-$C_6H_2$- | -$C(CH_3)_3$ [Fumarat] | 173-174 |
| 50 | 4-$CH_3CO$, 3-$CH_3S$-$C_6H_3$- | -$C(CH_3)_3$ | ($F_f$-Wert auf DC Kieselgel F 254, Schichtdicke 0,2 mm, Fließmittel Toluol Ethanol = 3:1 = 0,365 |
| 51 | 4-OH, 3-$HOCH_2$-$C_6H_3$- | -$C(CH_3)_3$ | Öl |
| 52 | 4-OH, 3-$CH_3SO_2NH$-$C_6H_3$- | i-Propyl | 135 |
| 53 | 2,5-$(CH_3O)_2$ $C_6H_3$- | t-Butyl | Öl |

Herstellung der Ausgangsprodukte

Beispiel a1

N-1,3,3-Trimethyl-propyl-phenylglyoxalaldonitron

$$C_6H_5-CO-CH=\overset{\overset{O^{\ominus}}{|}}{N^{\oplus}}-CH(CH_3)C(CH_3)_3$$

5 g 3,3-Dimethyl-2-butyl-hydroxylamin HCl, 2,6 g Natriumacetat und 5,7 g Phenylglyoxalhydrat wurden in 100 ml Ethanol 2,5 Stunden bei Raumtemperatur gerührt. Man filtrierte, engte das Filtrat bis auf 10 ml ein und versetzte es mit 20 ml Wasser. Nach einer Stunde unter Kühlung auf 1 °C wurde der entstehende Niederschlag abfiltriert und aus Methanol/Wasser umkristallisiert.

Ausbeute : 5 g. Schmp. : 134-135 °C.

Analog wurden folgende Verbindungen der Formel II erhalten :

| Beispiel | Ar | R | Schmp.: |
|---|---|---|---|
| a2 | $C_6H_5$ | $-CH(CH_3)_2$ | 92-93° C |
| a3 | $C_6H_5$ | $-CH_3$ | 90-94° C |
| a4 | $C_6H_5$ | $-C(CH_3)_3$ | 79° C |
| a5 | $4-NH_2,3,5-Cl_2-C_6H_2$ | $-CH_3$ | 122° C |
| a6 | $4-NH_2,3,5-Cl_2-C_6H_2$ | $-C(CH_3)_3$ | 173° C Zers. |
| a7 | $4-NH_2,3,5-Cl_2-C_6H_2$ | $-CH(CH_3)_2$ | 155° C Zers. |
| a8 | $4-NH_2,3,5-Cl_2-C_6H_2$ | $-C(CH_3)_2C\equiv CH$ | 113-120° C Zers. |
| a9 | $4-NH_2,3,5-Cl_2-C_6H_2$ | $-C(CH_3)_2C_2H_5$ | 118-120° C |
| a10 | $4-NH_2,3,5-Cl_2-C_6H_2$ | $-CH(CH_3)-CH_2-2-F-C_6H_4$ | Oel |
| a11 | $4-NH_2,3,5-Cl_2-C_6H_2$ | $-CH(CH_3)-CH_2-3-CF_3-C_6H_4$ | Oel |
| a12 | $4-NH_2,3,5-Cl_2-C_6H_2$ | $-CH(CH_3)-CH_2-2-CH_3O-C_6H_4$ | Oel |
| a13 | $4-NH_2,3,5-Cl_2-C_6H_2$ | $-CH(CH_3)-CH_2-3,4-CH_3O-C_6H_3$ | Oel |
| a14 | $4-NH_2,3,5-Cl_2-C_6H_2$ | $-CH(CH_3)-CH_2-4-CH_3O-C_6H_4$ | Oel |
| a15 | $4-NH_2,3,5-Cl_2-C_6H_2$ | $-C(CH_3)_2-CH_2OH$ | Oel |
| a16 | $4-NH_2,3,5-Cl_2-C_6H_2$ | $-CH(cyclo-C_3H_5)_2$ | Oel |
| a17 | $4-NH_2,3,5-Cl_2-C_6H_2$ | $-CH(CH_3)(CF_3)$ | Oel |
| a18 | $4-NH_2,3,5-Cl_2-C_6H_2$ | $-CH_2CF_3$ | Oel |
| a19 | $4-NH_2,3,5-Cl_2-C_6H_2$ | $-CH_2-C(CH_3)_3$ | Oel |
| a20 | $4-NH_2,3,5-Cl_2-C_6H_2$ | $-CH_2-CH(CH_3)_2$ | Oel |
| a21 | $4-NH_2,3,5-Cl_2-C_6H_2$ | $-CH(CH_3)CH_2-4-F-C_6H_4$ | Oel |
| a22 | $4-NH_2,3,5-Cl_2-C_6H_2$ | $-CH(CH_3)-CH_2-2-Cl,6-F-C_6H_3$ | Oel |
| a23 | $4-NH_2,3,5-Cl_2-C_6H_2$ | $-CH_2-CH_2-3,4-CH_3O-C_6H_4$ | Oel |

Beispiel b

Die Hydroxylamine gemäß Formel IV der folgenden Tabelle wurden nach bekannten Verfahren hergestellt.

Verfahren a) = W. D. Emmons, J. Amer. Chem. Soc. 79, 5739-54, (1957)

Verfahren b) = R. Borch, J. Am. Chem. Soc. 93, 2897, (1971)

| Bsp. Nr. | Formel | Verfahren | phys.Daten |
|---|---|---|---|
| b1 | 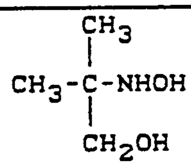 | a) | |
| b2 | 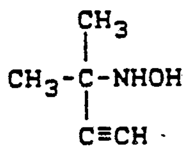 | a) | |
| b3 | 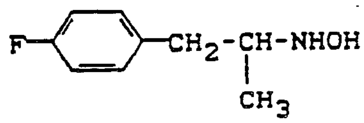 | b) | |
| b4 | 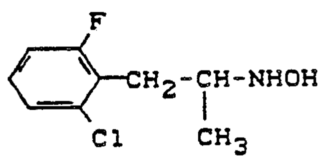 | b) | |
| b5 | 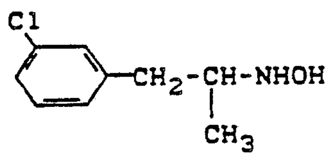 | b) | |
| b6 | 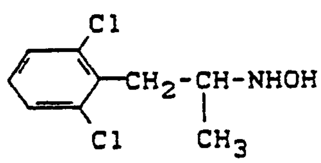 | b) | |
| b7 | 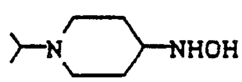 | b) | |
| b8 | 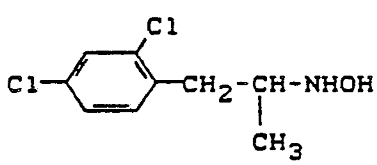 | b) | |
| b9 | 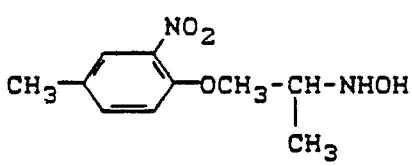 | b) | |
| b10 | 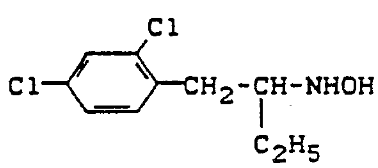 | b) | |
| b11 | 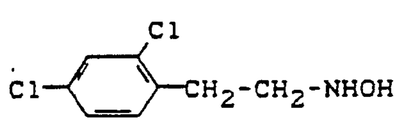 | b) | |

(Fortsetzung)

| Bsp. Nr. | Formel | Verfahren | phys.Daten |
|---|---|---|---|
| b12 | 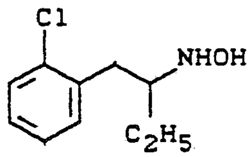 | b) | |
| b13 | 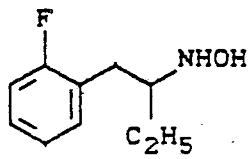 | b) | |
| b14 | 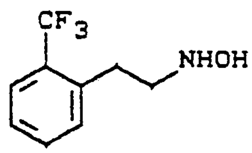 | b) | |
| b15 | 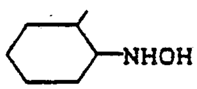 | b) | |
| b16 | $C(_2H_5)_2$-CH-NHOH | b) | |
| b17 | $C(H_3)_2CH$-$CHCCH_3$)-NHOH | b) | |
| b18 | $CH_3(CH_2OH)$-CHNHOH | b) | |
| b19 | $(nC_3H_7)CH_3$-CHNHOH | b) | |
| b20 | $((CH_3)_2CH)$-$CH_3$-CHNHOH | b) | |
| b21 | $(C_2H_5)_2$-CHNHOH | b) | |

**Patentansprüche**

1. Arylethanolhydroxylamine der Formel I

$$\mathrm{Ar} - \overset{\displaystyle OH}{\overset{|}{CH}} - CH_2 - \overset{\displaystyle OH}{\overset{|}{N}} - R \qquad (I)$$

in welcher

Ar für Aryl, das gegebenenfalls substituiert sein kann, steht,

R für Alkyl, Aryl, Aralkyl, Heteroaryl, Heteroaralkyl, Cycloalkyl, die gegebenenfalls substituiert sein können, steht,

sowie deren physiologisch verträgliche Salze, Enantiomeren und Diastereomeren.

2. Verfahren zur Herstellung der Arylethanolhydroxylamine der Formel I

$$\mathrm{Ar} - \overset{\displaystyle OH}{\overset{|}{CH}} - CH_2 - \overset{\displaystyle OH}{\overset{|}{N}} - R \qquad (I)$$

in welcher

Ar für Aryl, das gegebenenfalls substituiert sein kann, steht,

R für Alkyl, Aryl, Aralkyl, Heteroaryl, Heteroaralkyl, Cycloalkyl, die gegebenenfalls substituiert sein können, steht,

dadurch gekennzeichnet, daß man Arylglyoxalnitrone der Formel II

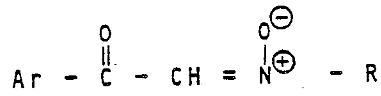
(II)

in welcher

Ar und R die oben angegebene Bedeutung haben, reduziert.

3. Arylglyoxalnitrone der Formel II

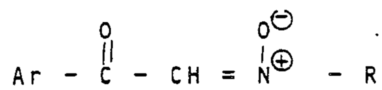
(II)

in welcher

Ar für Aryl, das gegebenenfalls substituiert sein kann, mit Ausnahme von Phenyl und p-Bromphenyl steht und

R für Alkyl, Aralkyl, Heteroaralkyl, Cycloalkyl, die gegebenenfalls substituiert sein können, steht.

4. Verfahren zur Herstellung der neuen Arylglyoxalnitrone der Formel II

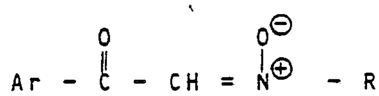
(II)

in welcher

Ar für Aryl, das gegebenenfalls substituiert sein kann, mit Ausnahme von Phenyl und p-Bromphenyl steht und

R für Alkyl, Aralkyl, Heteroaralkyl, Cycloalkyl, die gegebenenfalls substituiert sein können, steht, dadurch gekennzeichnet, daß man Arylglyoxale der Formel III

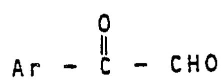
(III)

in welcher

Ar die oben angegebene Bedeutung, hat, gegebenenfalls in Form ihrer Hydrate oder Addukte mit Alkoholen mit Hydroxylaminen der Formel IV

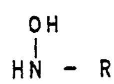
(IV)

in welcher

R die oben angegebene Bedeutung hat, unter Wasserabspaltung umsetzt.

5. Mittel zur Leistungsförderung von Tieren, gekennzeichnet durch einen Gehalt an Arylethanolhydroxylaminen der Formel I gemäß Anspruch 1.

6. Tierfutter, Trinkwasser für Tiere, Zusätze für Tierfutter und Trinkwasser für Tiere, gekennzeichnet durch einen Gehalt an Arylethanolhydroxylaminen der Formel I gemäß Anspruch 1.

7. Verwendung von Arylethanolhydroxylaminen der Formel I gemäß Anspruch 1 zur Leistungsförderung von Tieren.

8. Verfahren zur Herstellung von Mitteln zur Leistungsförderung von Tieren, dadurch gekennzeichnet, daß man Arylethanolhydroxylamine der Formel I gemäß Anspruch 1 mit Streck- und/oder Verdünnungsmitteln versetzt.

9. Verfahren zur Herstellung von Tierfutter, Trinkwasser für Tiere oder Zusätze für Tierfutter und Trinkwasser für Tiere, dadurch gekennzeichnet, daß man Arylethanolhydroxylamine der Formel I gemäß Anspruch 1 mit Futtermitteln oder Trinkwasser und gegebenenfalls weiteren Hilfsstoffen vermischt.

10. N-2-(4-Amino-3,5-dichlor-phenyl)-2-hydroxy-ethyl-N-t-butyl-hydroxylamin.

## Claims

1. Arylethanol-hydroxylamines of the formula I

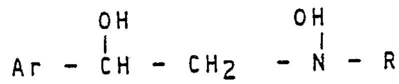

(I)

in which

Ar represents aryl which can optionally be substituted, and

R represents alkyl, aryl, aralkyl, heteroaryl, heteroaralkyl or cycloalkyl, each of which can optionally be substituted,

and their physiologically tolerated salts, enantiomers and diastereomers.

2. Process for the preparation of the arylethanol-hydroxylamines of the formula I

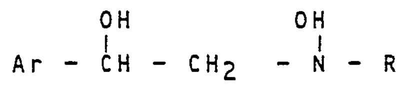

(I)

in which

Ar represents aryl which can optionally be substituted, and

R represents alkyl, aryl, aralkyl, heteroaryl, heteroaralkyl or cycloalkyl, each of which can optionally be substituted,

characterized in that arylglyoxal nitrones of the formula II

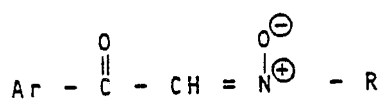

(II)

in which

Ar and R have the abovementioned meaning, are reduced.

3. Arylglyoxal nitrones of the formula II

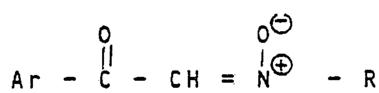

(II)

in which

Ar represents aryl which can optionally be substituted, with the exception of phenyl and p-bromophenyl, and

R represents alkyl, aralkyl, heteroaralkyl or cycloalkyl, each of which can optionally be substituted.

4. Process for the preparation of the new arylglyoxal nitrones of the formula II

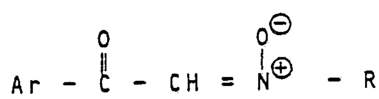

(II)

in which

Ar represents aryl which can optionally be substituted, with the exception of phenyl and p-bromophenyl and

R represents alkyl, aralkyl, heteroaralkyl or cycloalkyl, each of which can optionally be substituted,

characterized in that arylglyoxals of the formula III

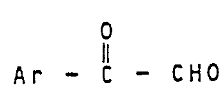

(III)

in which

Ar has the abovementioned meaning, where appropriate in the form of their hydrates or adducts with alcohols, are reacted with hydroxylamines of the formula IV

$$H\overset{\overset{OH}{|}}{N} - R \qquad (IV)$$

in which

R has the abovementioned meaning, with elimination of water.

5. Agents for the production promotion of livestock, characterized by containing arylethanol-hydroxylamines of the formula I according to Claim 1.

6. Animal feed, drinking water for livestock, additives to animal feed and drinking water for livestock, characterized by containing arylethanol-hydroxylamines of the formula I according to Claim 1.

7. Use of arylethanol-hydroxylamines of the formula I according to Claim 1 for the production promotion of livestock.

8. Process for the preparation of agents for the production promotion of livestock, characterized in that arylethanol-hydroxylamines of the formula I according to Claim 1 are mixed with extenders and/or diluents.

9. Process for the preparation of animal feed, drinking water for livestock or additives to animal feed and drinking water for livestock, characterized in that arylethanol-hydroxylamines of the formula I according to Claim 1 are mixed with feedstuffs or drinking water and, where appropriate, further auxiliaries.

10. N-2-(4-amino-3,5-dichloro-phenyl)-2-hydroxy-ethyl-N-t-butyl-hydroxylamine.

**Revendications**

1. Aryléthanolhydroxylamines répondant à la formule I

$$Ar - \overset{\overset{OH}{|}}{C}H - CH_2 - \overset{\overset{OH}{|}}{N} - R \qquad (I)$$

dans laquelle

Ar représente un groupe aryle pouvant éventuellement être substitué,

R représente un groupe alkyle, aryle, aralkyle, hétéroaryle, hétéroaralkyle, cycloalkyle, qui peuvent être éventuellement substitués,

ainsi que leurs sels physiologiquement compatibles, leurs énantiomères et leurs diastéréomères.

2. Procédé de production des aryléthanolhydroxylamines répondant à la formule I

$$Ar - \overset{\overset{OH}{|}}{C}H - CH_2 - \overset{\overset{OH}{|}}{N} - R \qquad (I)$$

dans laquelle

Ar représente un groupe aryle pouvant éventuellement être substitué

R représente un groupe alkyle, aryle, aralkyle, hétéroalkyle, hétéroaralkyle, cycloaryle qui peuvent être éventuellement substitués,

caractérisé en ce que l'on réduit les arylglyoxalnitrones répondant à la formule II

$$Ar - \overset{\overset{O}{\|}}{C} - CH = \overset{\overset{O^{\ominus}}{|}}{N^{\oplus}} - R \qquad (II)$$

dans laquelle

Ar et R ont les significations mentionnées ci-dessus.

3. Arylglyoxalnitrone répondant à la formule II

$$Ar - \overset{\overset{O}{\|}}{C} - CH = \overset{\overset{O^{\ominus}}{|}}{N^{\oplus}} - R \qquad (II)$$

dans laquelle

Ar représente un groupe aryle pouvant éventuellement être substitué, à l'exception des groupes phényle et p-bromophényle

R représente un groupe alkyle, aralkyle, hétéroalkyle, cycloalkyle, qui peuvent éventuellement être substitués.

4. Procédé de production des nouvelles arylglyoxalnitrones répondant à la figure II

$$Ar - C(=O) - CH = N^{\oplus}(O^{\ominus}) - R \qquad (II)$$

dans laquelle

Ar représente un groupe aryle pouvant éventuellement être substitué, à l'exception des groupes phényle et p-bromophényle et

R représente un groupe alkyle, aralkyle, hétéroalkyle, cycloalkyle, qui peuvent éventuellement être substitués,

caractérisé en ce que l'on convertit les arylglyoxals répondant à la formule III

$$Ar - C(=O) - CHO \qquad (III)$$

dans laquelle

Ar a la signification mentionnée ci-dessus, éventuellement sous forme de leurs hydrates ou composés d'addition, avec des alcools avec des hydroxylamines répondant à la formule IV

$$HN(OH) - R \qquad (IV)$$

dans laquelle

R a la signification ci-dessus, avec élimination d'eau.

5. Agent de stimulation du rendement des animaux, caractérisé par une teneur en aryléthanolhydroxylamines répondant à la formule I, selon la revendication 1.

6. Aliments pour animaux, eau potable pour animaux, additifs pour aliments pour animaux et eau potable pour animaux, caractérisés par une teneur en aryléthanolhydroxylamines répondant à la formule I et selon revendication 1.

7. Utilisation des aryléthanolhydroxylamines répondant à la formule I selon la revendication 1, pour stimuler le rendement des animaux.

8. Procédé de production d'agents stimulant le rendement des animaux, caractérisé en ce que l'on traite les aryléthanolhydroxylamines répondant à la formule I et selon la revendication 1 au moyen d'agents de dilution et/ou d'allongement.

9. Procédé de production d'aliments pour animaux d'eau potable pour animaux ou d'additifs pour aliments pour animaux et eau potable pour animaux, caractérisé en ce que l'on mélange des aryléthanolhydroxylamines répondant à la formule I et selon revendication 1 avec des aliments ou de l'eau potable et, éventuellement, d'autres adjuvants.

10. N-2-(4-amino-3,5-dichloro-phényl)-2-hydroxyéthyl-n-t-butyl-hydroxylamine.